(19) Europäisches Patentamt European Patent Office Office européen des brevets

(11) **EP 4 649 892 A1**

(12) **EUROPEAN PATENT APPLICATION**

(43) Date of publication:
**19.11.2025 Bulletin 2025/47**

(21) Application number: **24175594.1**

(22) Date of filing: **14.05.2024**

(51) International Patent Classification (IPC):
**A61B 8/04** (2006.01)   **A61B 8/06** (2006.01)
**A61B 8/08** (2006.01)   **A61B 8/00** (2006.01)

(52) Cooperative Patent Classification (CPC):
**A61B 8/04; A61B 8/06; A61B 8/0883;**
**A61B 8/4236; A61B 8/488; A61B 8/5223;**
A61B 8/02; A61B 8/065; A61B 8/0891;
A61B 8/4227; A61B 8/4477; A61B 8/46;
A61B 8/5246

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• SHULEPOV, Sergey Yuryevich
  Eindhoven (NL)
• JOSHI, Rohan
  Eindhoven (NL)

(74) Representative: **Philips Intellectual Property &**
**Standards**
**High Tech Campus 52**
**5656 AG Eindhoven (NL)**

(54) **METHOD FOR HEMODYNAMIC MONITORING**

(57)   A method for detection of acute hemodynamic events via non-invasively acquired continuous arterial measurements of arterial diameter and arterial blood flow. From these a surrogate blood pressure waveform signal can be obtained. This can be used and/or the blood volume waveform can be used to monitor for occurrence of one or more types of acute hemodynamic event.

FIG. 2

EP 4 649 892 A1

## Description

FIELD OF THE INVENTION

This invention relates to a method for non-invasive hemodynamic monitoring.

BACKGROUND OF THE INVENTION

Continuous hemodynamic monitoring is an important aspect of patient management in pre/peri/postoperative settings. Traditional vital signs, however, such as heart rate (HR) and blood pressure (BP) may be deceptively normal, while acute clinical events, e.g. large blood loss, acute aortic valve regurgitation, and other events, may still occur.

Physical symptoms of acute events, such as bleeding (hemorrhage), depend on location and are not always overtly apparent. Thus, these cannot be relied upon as an indicator of acute event onset.

The gold standard for hemodynamic monitoring is an invasive arterial catheter. This allows for obtaining a continuous measurement of the blood pressure (BP) waveform. However, due to central autoregulation, even this signal may not reliably reflect the patient status at the beginning of an acute event. Moreover, invasive catheter use carries risk of nosocomial infections.

By way of example, in the case of gastrointestinal (GI) bleeding, traditional monitoring involving vital signs such as HR and BP may be deceptively close to normal in the early stages of bleeding. In fact, blood pressure may even not drop until 750 to 1500 mL of blood is lost, i.e., in a mild-to-moderate hypovolemia.

By way of one further example, in the case of acute regurgitation of heart valves, it is known that, in some patients, an almost instantaneous ventricular adaptation may take place which results in a nearly normal response in traditional vital signs.

From a clinical perspective, it is important that occurrence of acute events is identified to a caregiver even when traditional vital signs appear stable. Thus, an improved method for non-invasive monitoring of hemodynamic status in a manner that permits more accurate detection of acute clinical events would be of value.

SUMMARY OF THE INVENTION

The invention is defined by the claims.

In accordance with an aspect of the invention, there is provided a hemodynamic monitoring method.

The method comprises receiving from a wearable ultrasound sensor real-time Doppler ultrasound data and real time B-mode ultrasound data corresponding to an artery.

The method may further comprise deriving a continuous or real-time blood velocity signal by processing of the Doppler ultrasound data.

The method may further comprise deriving, by processing of the B-mode ultrasound data, a real-time arterial diameter signal indicative of real time arterial diameter or a proxy or correlate thereof.

The method may further comprise computing a real-time blood flow or blood volume waveform based on the arterial diameter signal and blood velocity signal.

The method may further comprise computing a continuous surrogate blood pressure waveform based on applying a pre-determined blood pressure transfer function to the arterial diameter signal.

The method further comprises detecting occurrence of one or more pre-defined acute hemodynamic events based on the continuous blood flow or blood volume waveform and/or the continuous surrogate BP waveform.

Thus, it is proposed according to embodiments of the present invention to use an ultrasound sensor/array, e.g. a wearable ultrasound sensor, to continuously obtain blood velocity, diameter, and/or flow measurements in real time from an arterial location and infer hemodynamic changes based thereon.

Due to continuous monitoring of multiple parameters at the same location, with a relatively high spatial and temporal resolution, fast changes can be captured, and acute events can be identified and separated from local and central autoregulation.

Ultrasound is non-invasive and thus is suitable for use in continuous monitoring. Furthermore, reductions in scale of ultrasound transducers has meant that ultrasound sensing can be integrated into small form factor units which are capable of being worn or attached to the body. For example, ultrasound patches are a possible implementation.

Embodiments of the invention allow for early identification of acute hemodynamic events, and also may allow for classification of the severity of a detected event. If a hemodynamic event is detected, a user-perceptible indicator of this may be communicated to a clinician user via a user interface, e.g. by display of an indicator on a display device, possibly along with a classification/estimation of the severity.

In some embodiments, a continuous blood flow signal, BF(t), is computed from the blood velocity signal, U(t), and the arterial diameter signal, D(t), based on the relationship:

$$BF(t) = C\pi D(t)^2 \cdot <U(t)>$$

where $<U(t)>$ is the time-average value of U(t) over a single heart cycle, and C is a constant. In some embodiments, the constant C may be set at a value of ½.

More generally, the constant C may be set as desired. In some embodiments, the constant C may be set in dependence upon the Womersley number (Wo) and type of pulsatile flow. In some embodiment, C may be set at a value of ½. This value is particularly applicable for example for laminar flow and low Wo number which is a most common scenario.

For example, $<U(t)> = \int_0^T U(t)\, dt$ where T is the period of a single heart cycle, or, for a discrete data series spanning a heart cycle, with N being the total number of samples over the cycle: $<U(t)> = \frac{1}{N} \sum_{N=0}^{N-1} U[n]$.

In some embodiments, the pre-determined blood pressure transfer function takes the following, linearized form:

$$p_{CCA}(t) = \delta D(t) + p_0$$

$$p_0 = p_d$$

$$\delta = \frac{MAP - p_d}{<D(t)>}$$

where $p_{CCA}(t)$ is an estimated pressure waveform in the common carotid artery, $D(t)$ is the derived arterial diameter signal, $p_d$ is a measured value of diastolic blood pressure, and $\delta$ is a scaling factor, wherein MAP is a measured value of mean arterial pressure and $<D(t)>$ is an average value of $D(t)$ over a single heart cycle.

Two main groups of embodiments are envisaged. According to one group of embodiments, detection of the acute events is achieved via signal decomposition of the surrogate blood pressure waveform and/or the continuous blood flow or blood volume waveform. According to a further group of embodiments, detection of acute events is achieved via using the blood volume signal and surrogate BP signal to compute a pressure-volume curve for a plurality of heart cycles and using this to monitor changes in cardiac work between heart cycles. The changes in cardiac work can be translated into detection of hemodynamic events.

The first group of embodiments will be discussed first.

According to one or more embodiments, the detecting one or more acute events may comprise applying a signal decomposition to one or both of the blood flow or volume waveform signal and the surrogate blood pressure waveform signal to derive for one or both waveforms a set of extracted signal components. The detecting the one or more acute events may be perform based on analysis of the extracted signal components for each waveform.

A decomposition of a signal allows for selecting components which are most representative of clinically relevant phenomena. The method may comprise tracking or monitoring changes in one or more of the components over time to detect trends in one or more components. The method may comprise tracking or monitoring changes in a difference between a certain same component of the blood flow/volume signal and the diameter or blood pressure signal. For example, the method may comprise tracking a phase difference between a certain same component of the blood flow/volume signal and the diameter or blood pressure signal. For example, detection of an adverse event may comprise detecting such a phase difference tending toward zero or reaching a certain proximity of zero.

By way of example, the signal decomposition may comprise an empirical mode decomposition (EMD). This technique is particularly useful for non-linear and non-stationary signals. Empirical mode decomposition decomposes a signal into Intrinsic Mode Functions (IMFs). The IMFs are functions with distinct time scales and well-defined local frequencies, and the decomposition is based on the local time-scale properties of the signal. The decomposition process is adaptive, meaning it does not rely on a predetermined basis set, unlike methods such as Fourier Transform.

However, other types of signal decomposition can also be considered, including for example wavelet decomposition, empirical wavelet transform (EWT), continuous or discrete wavelet transform (CWT or DWT).

In some embodiments, Multi Resolution Analysis (MRA) may be performed to extract behavior/trending of the volume and/or blood pressure signals in time, for example based on detecting trending in one or more of the decomposed components of the volume and/or blood pressure signals.

Further alternatives include for example Principal Component Analysis (PCA); Singular Spectrum Analysis (SSA): Mode Decomposition Methods such as Proper Orthogonal Decomposition (POD) or Dynamic Mode Decomposition (DMD); Fourier Decomposition.

In all cases, preferably the chosen signal decomposition technique is a phase-preserving decomposition.

In some embodiments, the detecting acute events comprises: detecting suspected acute events by analysis of the extracted signal components of the continuous blood flow or blood volume waveform and the continuous surrogate BP waveform; computing one or more cardiovascular indices using said waveforms for signal time periods corresponding to the suspected acute events; and confirming suspected acute events as confirmed acute events based on comparison of the computed indices against pre-defined confirmation criteria. For example, the confirmation criteria might include a threshold for one or more of the cardiovascular indices.

By way of non-limiting example, the computed indices may include one or more of: heart rate (HR); carotid flow time (CFT); corrected carotid flow time (CFTc); pulse contour (pressure); velocity time integral (VTI); and phase shift between flow and pressure features ($\Delta$Phase).

As mentioned above, according to a further group of embodiments, detection of acute events is achieved via using the blood volume signal and surrogate BP signal to compute a pressure-volume (PV) curve for a plurality of heart cycles and using this to monitor changes in cardiac work between heart cycles. The changes in cardiac work can be translated into detection of hemodynamic events.

For example, according to this set of embodiments, PV loops or curves are used to estimate real time measure of heart work done, and detect acute events based on changes in heart work done which are not accompanied by meaningful changes in blood pressure or other vitals.

In accordance with this set of embodiments, the method may comprise, at repeated time points during a patient monitoring session:

> deriving a single-pulse blood volume waveform based on the computed real-time blood volume signal;
>
> deriving a single-pulse blood pressure waveform based on the computed real-time blood pressure signal;
>
> deriving a Pressure-Volume curve over a single pulse cycle based on the single-pulse blood volume waveform and the single-pulse blood pressure waveform;
>
> deriving a measure of cardiac work based on computing an area under the Pressure-Volume curve, and recording the measure of cardiac work in a cardiac work data series for the monitoring session; and

detecting acute cardiovascular events based on detecting changes in the measure of cardiac work during the monitoring session which meet one or more pre-defined alert criteria.

In some embodiments, the deriving the single-pulse blood volume waveform based on the real-time blood volume signal may comprise computing an average blood volume waveform from a plurality of pulse waveforms in the real-time blood volume signal.

In some embodiments, the deriving the single-pulse blood pressure waveform based on the real-time blood pressure signal may comprise computing an average blood pressure waveform from a plurality of pulse waveforms in the real-time blood pressure signal.

In some embodiments, the method may further comprise estimating a phase offset between the single-pulse blood volume waveform and the single pulse blood pressure waveform by determining an average phase offset between peaks of a plurality of pulses in the real-time blood velocity signal and peaks of a plurality of pulses in the real-time arterial diameter signal. This exploits the fact that the velocity acts like the volume and diameter acts like BP.

The method may comprise time-aligning the single-pulse blood volume waveform and single-pulse blood pressure waveform based on the phase offset.

According to any of the above-described embodiments, the artery may be a peripheral artery.

Another aspect of the invention is a computer program product comprising computer program code configured, when run on a processor, to cause the processor to perform a method in accordance with any preceding claim.

Another aspect of the invention is a processing device, comprising an input/output for connecting to a wearable ultrasound sensor, and one or more processors, configured to perform a method in accordance with any embodiment described in this disclosure or in accordance with any claim of this application.

Another aspect of the invention is a system comprising: a wearable ultrasound sensor; and a processing device as recited above. The wearable ultrasound sensor may comprise an ultrasound transducer array in some embodiments.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Fig. 1 outlines steps of an example method in accordance with one or more embodiments of the invention;
>
> Fig. 2 is a block diagram of an example processing device and system in accordance with one or more embodiments of the invention;
>
> Fig. 3 shows how an acute cardiac event may manifest in a blood pressure and blood flow signal;
>
> Fig. 4 shows how an acute cardiac event may manifest in a mean arterial pressure signal;
>
> Fig. 5 illustrates deriving a single-pulse blood pressure waveform based on extracting beats from a blood pressure signal;
>
> Fig. 6 illustrates deriving an average beat from a blood pressure signal;
>
> Fig. 7 illustrates deriving an ensemble average blood volume waveform;
>
> Fig. 8 shows a time synchronized representation of an ECG signal, velocity signal and arterial diameter signal for a patient; and
>
> Fig. 9 shows an example Pressure-Volume (PV) curve over a single pulse cycle.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for detection of acute hemodynamic events via non-invasively acquired continuous arterial measurements of arterial diameter and arterial blood flow. From these, a surrogate blood pressure waveform signal can be obtained. This can be used and/or the blood volume waveform can be used to monitor for occurrence of one or more types of acute hemodynamic event.

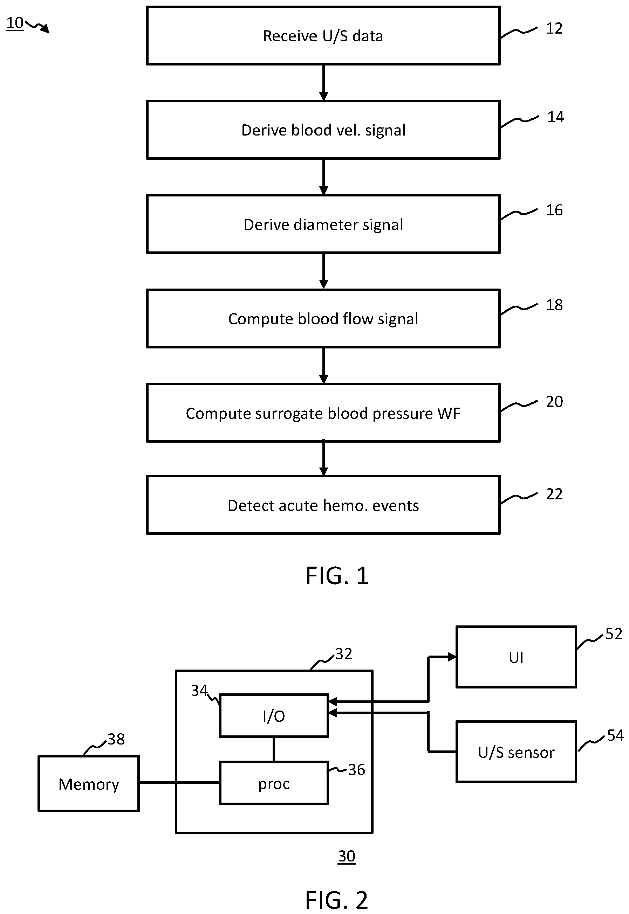

Fig. 1 outlines in block diagram form steps of an example method according to one or more embodiments. The steps will be recited in summary, before being explained further in the form of example embodiments.

Provided is a method for hemodynamic monitoring.

The method 10 comprises receiving 12, for example from a wearable ultrasound ("U/S") sensor, real-time Doppler ultrasound data and real time B-mode ultrasound data corresponding to an artery.

The method further comprises deriving 14 a continuous or real-time blood velocity signal by processing of the Doppler ultrasound data.

The method further comprises deriving 16, by processing of the B-mode ultrasound data, a real-time arterial diameter signal indicative of real time arterial diameter or a proxy thereof.

The method further comprises computing 18 a real-time blood flow or blood volume waveform based on the arterial diameter signal and blood velocity signal.

The method further comprises computing 20 a continuous surrogate blood pressure waveform based on applying a pre-determined blood pressure transfer function to the arterial diameter signal.

The method further comprises detecting 22 occurrence of one or more pre-defined acute hemodynamic events based on the continuous blood flow or blood volume waveform and the continuous surrogate BP waveform ("WF").

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises a user interface 52.

In the illustrated example of Fig. 2, the system 30 further comprises an ultrasound acquisition or sensing apparatus 54 for acquiring ultrasound data. For example, this may comprise a wearable ultrasound sensor. In some embodiments, the ultrasound sensor may comprise an ultrasound transducer array. In some embodiments, the ultrasound sensor may comprise a wearable ultrasound patch, meaning a patch which is affixable to the body and which carries one or more ultrasound transducers, for example an ultrasound transducer array.

The system 30 may further comprise a memory 38 for storing computer program code (i.e. computer-executable code) which is configured for causing the one or more processors 36 of the processing device 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

Embodiments of the invention involve the acquisition of blood velocity and arterial diameter measurements in an artery using the same single modality (ultrasound) and the inference therefrom of a continuous blood flow waveform (blood flow signal), and a surrogate of a blood pressure waveform (arterial blood pressure signal).

Analysis of these waveforms, e.g. through decomposition, allows for separation of acute clinical events from normal variability and/or possible errors in the acquisition. Based on these waveforms, different indices, for example indicating presence and/or severity of one or more acute clinical events can be constructed.

Instead of or in addition to decomposition, another approach is to estimate a real time work done by the heart by generating pressure-volume (PV) loops and identify periods where the work done has changed significantly without a commensurate change in one or more vital signs which are usually expected to correlate with work done.

For constructing the PV loops, the blood pressure signal can be acquired either through a direct measurement like an arterial line or estimated by using ultrasound. The arterial volume may be measured in an artery using ultrasound. Notable the PV loops are estimated at an arterial location (e.g., carotid) and not the heart (which requires invasive catheterization to measure pressure and cardiac ultrasound for volume).

More details regarding the acquisition of the ultrasound data signals will now be presented.

In accordance with one or more embodiments, the method comprises using an ultrasound transducer array, e.g. a wearable ultrasound device, to continuously acquire an arterial diameter (D) measurement signal via B-mode acquisition and simultaneously continuously acquire a local blood velocity (*U*) measurement signal via Doppler ultrasound acquisition, e.g. pulsed wave Doppler (PWD) and/or color Doppler.

These D and U measurements can be used to construct blood flow, BF, and effective blood pressure, BP, waveforms:

$$BF(t) = C\pi D(t)^2 \cdot < U(t) >$$

$$BP(t) = TF[D(t)]$$

where $< U(t) >$ is the time-average value of U(t) over a single heart cycle, TF is pre-defined blood pressure transfer function configured to transform an arterial diameter signal, or a correlate thereof, to an estimated arterial blood pressure signal, and C is a constant.

In some embodiments, the constant C may be set at a value of ½.

More generally, the constant C may be set as desired. In some embodiments, the constant C may be set in dependence upon the Womersley number (Wo) and type of pulsatile flow. In some embodiment, C may be set at a value of ½. This value is particularly applicable for example for laminar flow and low Wo number which is a most common scenario.

With regards to $< U(t) >$, for example, this may be computed as:

$$< U(t) > = \int_0^T U(t)\, dt$$

where T is the period of a single heart cycle, or, for a discrete data series spanning a heart cycle, with N being the total number of samples over the cycle:

$$< U(t) > = \frac{1}{N}\sum_{N=0}^{N-1} U[n]$$

With regards to the pre-defined transfer function, TF, this may take the following form:

$$p_{CCA}(t) = \delta D(t) + p_0$$

$$p_0 = p_d$$

$$\delta = \frac{MAP - p_d}{< D(t) >}$$

where pccv(t) is an estimated blood pressure waveform in the common carotid artery, $D(t)$ is the derived arterial diameter signal, $p_d$ is a measured value of diastolic blood pressure, and $\delta$ is a scaling factor, wherein MAP is a measured value of mean arterial pressure and $< D(t) >$ is an average value of $D(t)$ over a single heart cycle.

Thus, the surrogate pressure waveform may be computed as a linear scaling of the diameter waveform. However, other options for the transfer function can also be considered, for example a transfer function incorporating an exponential relationship.

It is noted that, in addition to the blood pressure and blood flow or volume signals, other vital signs can be extracted from the ultrasound data, including for example any one or more of heart rate (HR) and heart rate variability, blood flow time (FT) and corrected flow time (FTc), and derivatives thereof. Any of these could additionally be used in detected acute hemodynamic events.

With regards to acquiring the ultrasound data, in some embodiments, the ultrasound acquisition apparatus may comprise a wearable ultrasound patch, for instance applied at the common carotid artery (CCA).

In some embodiments, an ultrasound device may be used suitable for acquiring triplex ultrasound data. Triplex ultrasound is a type of ultrasound acquisition that combines three different modes of ultrasound: B-mode, Color Doppler and Spectral Doppler. The spectral doppler may be pulsed wave doppler in some embodiments.

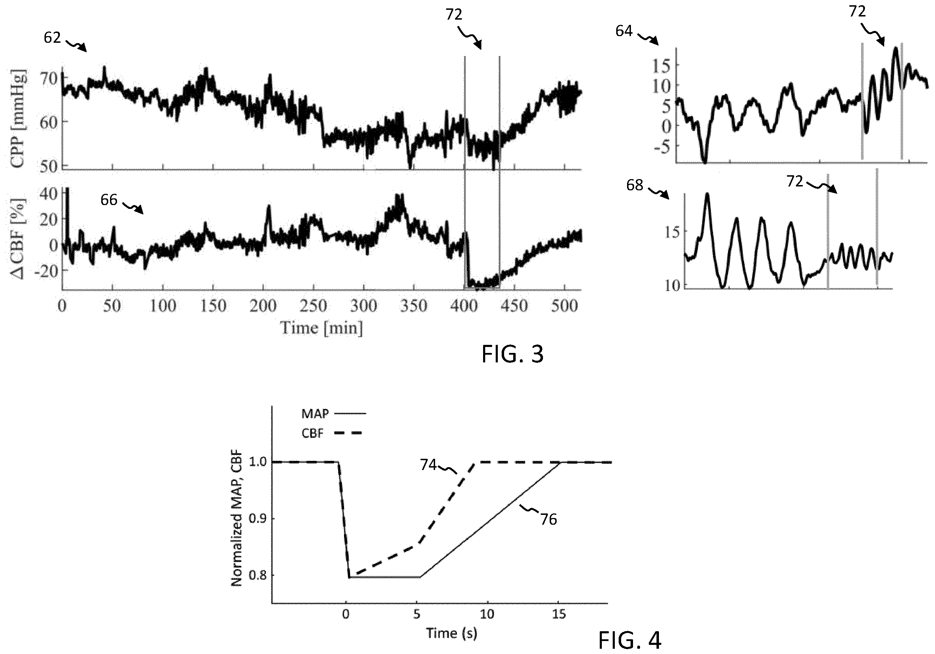

Fig. 3 illustrates how acute cardiac events may be reflected in real time arterial blood pressure and blood flow signals. Trace 62 shows Cerebral Perfusion Pressure (CPP), in mmHg, as a function of time and trace 66 shows change in Cerebral Blood Flow (ΔCBF), units %, as a function of time. An acute cardiovascular event occurs in the time period between the vertical grey lines, indicated by arrow 72. It can be seen that CPP and ΔCBF both drop suddenly.

Trace 64 shows a continuous arterial blood pressure signal spanning the same time period as trace 62 and trace 68 shows an arterial blood flow signal spanning the same period as trace 66. It can be seen that during the time period 72 in which the acute cardiovascular event occurs, a change in the periodic or spectral properties of both the blood pressure 64 and blood flow 68 signals occurs. Thus, it will be recognized that the blood pressure and blood flow or volume signals can be used to detect occurrence of acute hemodynamic events.

It is noted that acute hemodynamic events are also detectable in periodically measured averaged hemodynamic parameters, such as mean arterial pressure (MAP). However, the non-continuous nature of these measurements means that acute events can be missed where their presentation in hemodynamic parameters occurs between measurement times. As discussed previously, hemodynamic autoregulation means that detectable presentations of acute events in measurable parameters can be transitory.

An example is illustrated in Fig. 4. Line 74 shows normalized Cerebral Perfusion Pressure (CPP) as a function of time, while line 76 shows mean arterial pressure (MAP) as a function of time over the same time period. A clinical event occurs at time t=0. The CBF drops at this point and the MAP also drops proportionately. However, the CBP has recovered again to normal levels within 10 seconds and the MAP has recovered again within 15 seconds (there is phase lag in MAP with respect to blood flow). Thus it will be recognized that if MAP measurements are taken e.g. every 15 minutes, the very short time scale presentation of the acute event (i.e. approx. 15 seconds) is very likely to be missed, meaning that its occurrence will go unnoticed. By contrast, by acquiring continuous measurements at an arterial site of blood pressure and blood flow, occurrence of acute events can be more reliably detected.

One way to detect acute events in the blood pressure signal and the blood flow/volume signal would be to detect fluctuations or inflections in one or both of the signals of a certain magnitude. However, in continuously monitored signals, the data is inherently noisy and prone to outliers. Thus, there is a significant challenge in tuning the detection thresholds so that the transitory and sometimes subtle presentations of acute events in the signals are not missed, but also wherein false positive detections are minimized.

To address this challenge, according to one or more embodiments, the detecting one or more acute events may comprise applying a signal decomposition to one or both of the blood flow or volume waveform signal and the surrogate blood pressure waveform signal to derive for one or both waveforms a set of extracted signal components. The detecting the one or more acute events may be perform based on analysis of the extracted signal components for each waveform.

For example, according to at least one group of embodiments, it is proposed to perform a signal decomposition of the surrogate blood pressure and the blood flow/volume signals and use only a selected subset of one or more of the components to detect occurrence of acute events.

A decomposition of a signal allows for selecting components which are most representative of clinically relevant phenomena. By way of example, the method may comprise tracking or monitoring changes in one or more of the components over time to detect trends in one or more components. The method may comprise tracking or monitoring changes in a difference between a certain same component of the blood flow/volume signal and the diameter or blood pressure signal. For example, the method may comprise tracking a phase difference between a certain same component of the blood flow/volume signal and the diameter or blood pressure signal. For example, detection of an adverse event may comprise detecting such a phase difference tending toward zero or reaching a certain proximity of zero.

By way of example, the signal decomposition may comprise an empirical mode decomposition (EMD). Empirical mode decomposition decomposes a signal into Intrinsic Mode Functions (IMFs). The IMFs are functions with distinct time scales and well-defined local frequencies, and the decomposition is based on the local time-scale properties of the signal. The decomposition process is adaptive, meaning it does not rely on a predetermined basis set, unlike methods such as Fourier Transform. Instead, EMD works directly with the data to identify the intrinsic oscillatory modes by iteratively extracting the IMFs. This process involves identifying local extrema, connecting these points using envelopes, and then extracting detail from the signal by subtracting these envelopes. The result is a series of IMFs that capture different scales or frequencies present in the original signal, each representing a natural oscillation mode free from riding waves. The skilled person will know how to perform a EMD of a signal.

According to one set of embodiments, the method comprises applying empirical mode decomposition to each of the surrogate blood pressure signal and the blood flow/volume signal to extract a plurality of IMFs from each. Each IMF represents a mode of the respective signal from which it was extracted.

In some embodiments, a subset of the extracted modes/components is selected for use in detecting the one or more acute clinical events. In some embodiments, one of the extracted modes/components is selected for use in detecting the one or more acute clinical events. In some embodiments, the method comprises identifying an extracted mode/component having the strongest physiological signal. In other words, the method may comprise identifying a mode/component having the highest SNR. In other words, the method may comprise identifying a mode/component corresponding most closely to the underlying physiological signal in the original measurement, i.e. blood pressure or blood flow/volume.

Identification of the one of the modes having the strongest physiological signal may be an estimate. It may be done based on determining a spectral profile of each mode and identifying the mode having a spectral profile most closely matching a pre-determined expected spectral profile for the physiological signal (i.e. the blood pressure signal or the blood flow/volume waveform as appropriate).

The other modes or components may be discarded. For example, the other modes may represent noise or bias components or may represent only weaker representations of the underlying physiological signal.

The detecting the one or more acute hemodynamic events may then be performed based on analysis applied to the selected one (or more) of the extracted modes/components of each of the blood pressure and blood flow/volume signals.

In some embodiments, the detecting the one or more acute hemodynamic events may comprise detecting one or more pre-defined waveform features in the selected component/mode. For example, the one or more pre-defined waveform features might include a fluctuation of a threshold size.

In some embodiments, the detection of the one or more acute hemodynamic events may comprise determining a trend in one or more features or characteristics of the extracted components/modes. In some embodiments, the detection of the one or more acute hemodynamic events may comprise determining a trend in a phase of the extracted components/mode.

By way of example, in one set of embodiments, the method may comprise performing empirical mode decomposition of both the surrogate blood pressure signal and the blood flow/volume signal and selecting a one (or a subset) of the component modes for each signal. This may be a same component of mode for each of the signals, and/or it may be a respective component/mode for each signal which carries a strongest physiological signal. The method may further comprise determining a phase difference between the respective extracted components/modes of the two signals. The method may comprise detecting occurrence of an acute hemodynamic event based on the phase difference meeting one or more pre-defined detection criteria. The method may further comprise monitoring the phase difference over time to determine a trend in the phase difference. The method may comprise detecting occurrence of an acute hemodynamic event based on the trend in the phase difference meeting one or more pre-defined detection criteria. In some embodiments, the method may comprise detecting occurrence of one or more acute hemodynamic events based on detecting the phase difference tending to zero (for example trending to zero for at least a pre-defined threshold period of time) and/or coming within a pre-defined proximity of zero.

Identifying a trend where phase difference approaches zero could signal a reduction in dynamic arterial compliance, potentially indicating a risk of infarct or other cardiovascular issues. It is in general known that there is a phase difference between waveforms corresponding to the blood velocity/flow and diameter/pressure in the arteries. Some small changes in this phase difference are expected due to variations in the overall arterial compliance. However, when the phase difference is trending to zero over a long period of time, this would indicate almost no dynamic compliance, and as a consequence, can lead e.g. to infarct at certain clinical events.

In the case of empirical mode decomposition, determining the phase difference between the selected IMFs of the two signals may comprise performing a Hilbert transform of the selected IMF of each signal, computing an instantaneous phase of each Hilbert transformed IMF, and then calculating a phase difference.

It is noted that, although empirical mode decomposition is proposed in examples presented above, similar effects can be achieved by using other types of signal decomposition. Other examples include for instance empirical wavelet transform (EWT), or continuous/discrete wavelet transform (CWT/DWT).

The method may comprise detecting suspected acute hemodynamic events using the extracted signal components/modes of the blood pressure and/or blood flow/volume signals and then performing a further process to corroborate or confirm the suspected acute hemodynamic events. The confirmation may comprise computing one or more cardiovascular indices using the blood pressure and/or blood flow waveforms for signal time periods corresponding to the suspected acute events. The method may comprise confirming suspected acute events as confirmed acute events based on comparison of the computed indices against pre-defined confirmation criteria. For example, the confirmation criteria might include a threshold for one or more of the cardiovascular indices.

By way of example, the one or more cardiac features may include any of one or more of: heart rate (HR); carotid flow time (CFT); corrected carotid flow time (CFTc); pulse contour (pressure); velocity time integral (VTI); phase shift between flow and pressure features (ΔPhase). A combination of two or more of these indices could be used in some embodiments, to improve sensitivity of acute event detection..

By way of one example, for detecting acute upper gastrointestinal bleeding, CFT and CFTc are good differentiating indicators. In this regard, reference is made to the following paper: Karadadas,et al, Assessment of the carotid artery Doppler flow time in patients with acute upper gastrointestinal bleeding, Turkish Journal of Emergency Medicine - Volume 20, Issue 1, January-March 2020.

By way of a further example, an orthostatic drop in blood pressure ≥ 10 mm Hg combined with an increase in pulse rate ≥ 20 bpm is suggestive of hypovolemia.

Thus, it can be seen that combining local information, e.g. on BP, HR, ΔCFTc and/or other indices, the acute event detection may be greatly improved with respect to sensitivity.

By way of further example, similar indices may also be used as indicators of acute cardiac insufficiency. For example, a combination of, e.g., HR, BP, and ΔCFTc may be used to identify presence of acute cardiac insufficiency. In this regard, reference is made to the following paper: Folts et al, Coronary and Hemodynamic Effects of Temporary Acute Aortic Insufficiency in Intact Anesthetized Dogs, Circulation Research. Vol. 35. August 1974.

According to a further set of embodiments, the detection of the one or more acute hemodynamic events may comprise evaluating a real time measure of heart work done and detecting changes in work done which meet one or more detection criteria.

By way of background, Pressure-volume (PV) loops are used in cardiac physiology to understand the relationship between pressure in the ventricles and the volume of blood they contain during a cardiac cycle. Traditionally, pressure-volume (PV) loops are generated using cardiac data. The area under the curve of a PV loop corresponds to the work done by the heart.

PV loops are typically generated using data obtained from a catheter equipped with pressure and volume sensors that is inserted into the ventricle. The loop is plotted with ventricular volume on the x-axis and ventricular pressure on the y-axis. As the heart beats, it moves through a cycle of contraction and relaxation, generating a loop-like graph. The shape and position of the loop can vary based on the heart's condition and function.

In accordance with one or more embodiments of the present invention it is proposed to generate PV loops using the ultrasound data acquired at an arterial location. In this way, it is possible to obtain a close proxy for the work done by the heart, especially if measurements are made at arterial locations close to the heart. In particular, the surrogate blood pressure signal can be used as an indicator of P and the blood flow/volume signal can be used as an indicator of V. It is even possible to measure P and V in different arteries and still generate a PV loop which gives clinically meaningful estimates of work done.

In accordance with embodiments of the present invention, an approach is proposed wherein ensemble averaging of beat waveforms is performed to improve the SNR of PV loops, and thus of the running work done estimate.

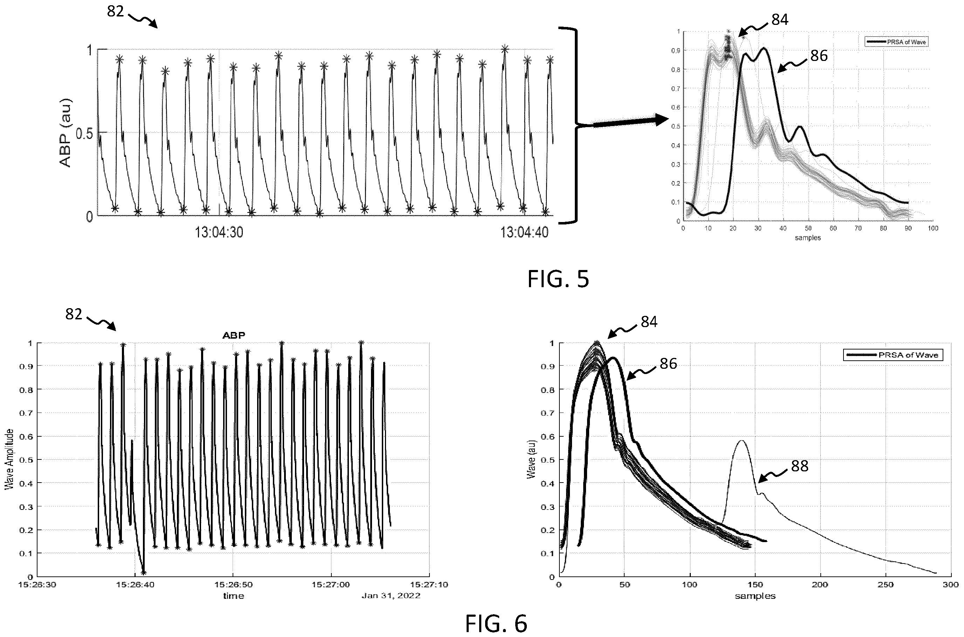

This is illustrated schematically in Fig. 5. Fig. 5 shows an example arterial pressure signal 82 derived using acquired ultrasound data as a surrogate signal in the manner discussed previously, or obtained using an invasive arterial line or using another non-invasive measurement method. The blood pressure signal spans a plurality of heart cycles and thus encompasses a plurality of pulses. In the illustrated example, the blood pressure signal spans an epoch of 30 seconds. The method further comprises deriving a single-pulse blood pressure waveform 84 based on the computed real-time blood pressure signal 82. This may for example comprise computing an average blood pressure waveform from a plurality of pulse waveforms in the real-time blood volume signal 82.

Computing the average blood pressure waveform may comprise generating an ensemble average or phase rectified signal average of at least a plurality of the pulse waveforms present in the blood pressure signal 82. This may comprise detecting the peaks and valleys in the arterial BP waveform 82 and extracting each beat in the signal, i.e., from valley to peak to valley. The extracted beats are shown as a group in Fig. 5, indicated by arrow 84. From the extracted beats, an averaged or rectified beat 86 centered around the peak of each heartbeat is generated.

Since each heartbeat is of a different exact time duration, in some embodiments, one can create a support for averaging based on, for instance the 75th percentile of the length from the peak to each of the valleys that encompass a heartbeat. This way, a robust BP waveform of a representative heartbeat can be generated, even if a peak or valley is missed. For example, this is illustrated in Fig. 6. Here, the blood pressure signal 82 includes an outlier beat 88 occurring after the third normal beat. Fig. 6 (right) shows the ensemble of extracted beats 84 from the blood pressure signal. The outlier beat 88 can also be seen, this displaced some way away from the rest of the beats. By using the 75th percentile of the length from the peak to each of the valleys that encompass a heartbeat as the support for averaging, the outlier values of this anomalous beat are excluded from the averaging. Fig. 6 shows the resulting averaged or rectified beat 86.

The average beat 86 in each case may be a phase rectified signal average (PRSA) beat.

The PRSA wave 86 has a phase delay. The approach to obtain this delay will be discussed in the description to follow.

A next stage in the procedure is to derive, using similar steps as were used to generate the single-pulse (average) pressure waveform, a single pulse (average) blood volume waveform.

There are two main ways to do this. One is to derive a single-pulse blood volume waveform based on the real-time blood volume signal computed from the blood velocity and arterial diameter signals. This may comprise computing an average of the pulse waveforms comprised by the blood volume signal. This can be done using the same or similar steps as were discussed above for deriving the average blood pressure waveform. Another approach is to use the blood velocity signal and the arterial diameter signal derived from the ultrasound data and to compute an average blood velocity waveform and an average arterial diameter signal for the same time period and to multiply the average blood velocity waveform with the average diameter waveform to yield an average blood volume pulse waveform. The average blood velocity and average arterial diameter waveforms can in some embodiments be derived using the same or similar steps as described above for deriving the average blood pressure waveform. The method may comprise deriving a phase rectified signal average of the blood volume pulse waveform in some embodiments.

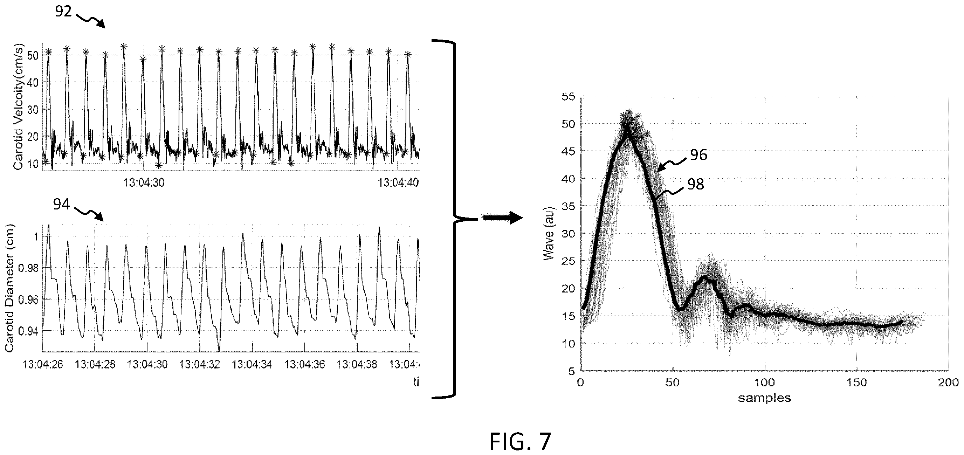

The deriving of an ensemble average volume waveform is illustrated schematically in Fig. 7. Fig. 7 illustrates an example blood velocity signal 92 derived from Doppler ultrasound data and an example diameter signal 94 derived from e.g. B-mode ultrasound data. The method may comprises taking a product of the velocity signal 92 and the diameter signal 94 to yield a set of blood volume or flow waveforms, indicated as an ensemble by arrow 96 in Fig. 7. In some embodiments, the method may comprise extracting each individual pulse waveform from the velocity signal 92 and extracting each individual pulse from the diameter signal 94 and then forming an ensemble of blood volume or flow waveforms by multiplying each of the pulse waveforms of the velocity signal with a temporally aligned or temporally corresponding pulse waveform of the diameter signal. This results in the ensemble of blood volume or blood flow pulse waveforms 96. From these, an ensemble average blood volume or flow pulse waveform 98 can be derived.

In some embodiments, the method may additionally comprise a procedure for estimating and correcting a phase or time offset between peaks/pulses of the velocity signal and peaks/pulses of the diameter signal.

It is known that, at any given arterial location, there is time offset between pressure and arterial diameter waves, due to the compliance of the artery wall.

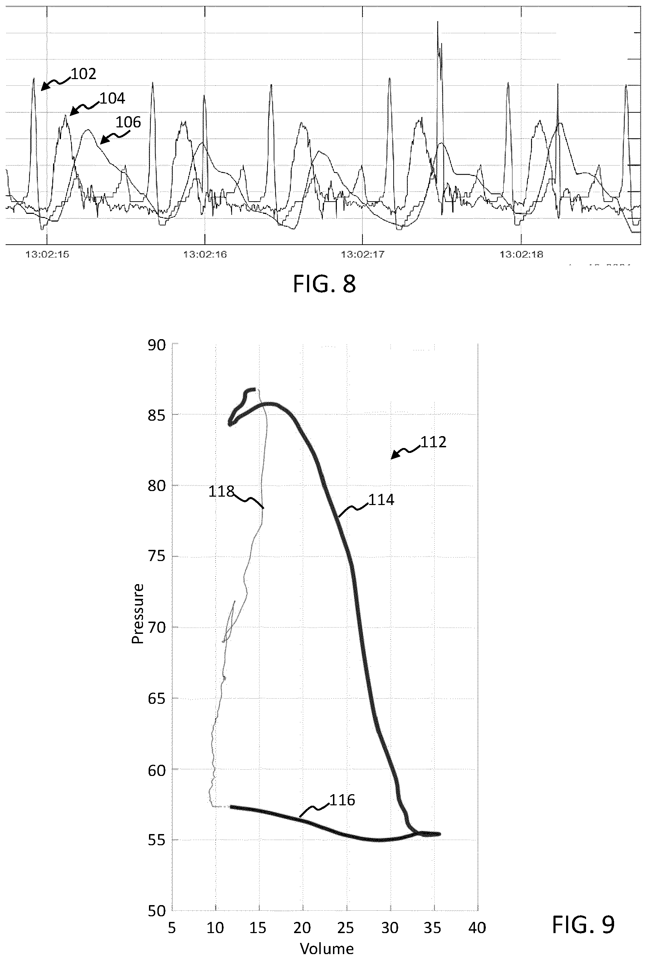

This is illustrated in Fig. 8 which shows a time synchronized representation of an ECG signal 102, velocity signal 104 and arterial diameter signal 106 for a patient over a time epoch of a few seconds which includes a plurality of pulses. Here, both the velocity signal 104 and the diameter signal 106 are acquired using ultrasound data of a same ultrasound device at a single arterial location. It can be seen that the ECG peak is first in time (its electrical nature means that it travels the fastest), followed by the velocity peak, followed by the diameter peak.

For purposes of computing the average blood flow waveform 98, there may be included a step of phase-aligning or time-aligning the peaks of the blood velocity signal with the peaks of the arterial diameter signal, so that corresponding peaks can be multiplied together in the manner discussed above.

To achieve this, in some embodiments, the method comprises calculating an average time difference or delay between the peaks of the velocity signal and of the diameter signal for multiple heartbeats (over a measurement epoch). This average time difference can be assumed to be the (physiological) delay between the blood pressure and the blood volume waves. This assumption is reasonable since it is well known that blood pressure and arterial diameter waves are closely aligned in time and it is also known that the measured velocity signal is a proxy for volume and the measured diameter signal is a proxy for blood pressure, at least for the purposes of time synchronization. Furthermore, the assumption also relies on the fact that the velocity and diameter waves are obtained from the same measurement dataset obtained using a same device at a single measurement location. Thus, there is no additional time delay to account for with respect to a positioning offset between measurement locations.

Using the average blood pressure waveform and the average blood volume waveform, the method may comprise deriving a Pressure-Volume (PV) curve over a single pulse cycle.

Fig. 9 shows an example PV curve 112 which may be generated. The x-axis represents blood volume (in mm), the y-axis represents arterial blood pressure (mmHg). A PV loop is generated by plotting arterial volume versus arterial pressure. The PV curve includes an arterial blood pressure upstroke 114, a blood volume upstroke 116 and an arterial blood pressure downstroke 118.

The PV loop 112 effectively acts as a proxy for cardiac work done. In particular, the area under the PV curve/loop can be understood as a measure of total cardiac work done over the course of a single heart cycle.

It is thus proposed to derive a measure of cardiac work based on computing an area under the Pressure-Volume curve, and recording the measure of cardiac work in a cardiac work data series for a monitoring session. In particular, it is proposed to derive a PV curve or loop recurrently or repeatedly over a monitoring session, derive a measure of cardiac work for each one, and then to track changes in cardiac work as a function of time. The method may then comprise detecting acute cardiovascular events based on detecting changes in the measure of cardiac work during the monitoring session which meet one or more pre-defined alert criteria.

The alert criteria may for example comprise detection of the cardiac work fluctuating by a threshold amount within a defined time window.

The alert criteria may comprise detecting a change in work done of a threshold amount while also not detecting a change in a further measurable patient parameter of a further threshold amount. In other words, the alert criteria might include detecting a threshold change in cardiac work done without detecting a change in a further parameter of a further threshold amount (measured over the same time period). For example, the further parameter could be blood pressure and/or one or more other vital signs. If work done changes (by a threshold amount) without a change in blood pressure (of a further threshold amount), this could indicate occurrence of an acute clinical event. This may trigger an alert for a clinical event.

In some embodiments, each constructed PV loop might be processed to extract one or more features of the PV loop, such as an average slope of the three segments 114, 116, 118 in Fig. 9.

By way of summary of the above-described process, there will now be outlined steps of an example method in accordance with one or more embodiments.

The method comprises:

receiving from a wearable ultrasound sensor real-time Doppler ultrasound data and real time B-mode ultrasound data corresponding to an artery;
deriving a continuous or real-time blood velocity signal by processing of the Doppler ultrasound data;
deriving, by processing of the B-mode ultrasound data, a real-time arterial diameter signal indicative of real time arterial diameter or a proxy thereof;

computing a real-time blood flow or blood volume signal based on the arterial diameter signal and blood velocity signal;

computing a continuous surrogate blood pressure signal based on applying a pre-determined blood pressure transfer function to the arterial diameter signal;

at repeated time points during a patient monitoring session:

deriving a single-pulse blood volume waveform based on the computed real-time blood volume signal;

deriving a single-pulse blood pressure waveform based on the computed real-time blood pressure signal;

deriving a Pressure-Volume curve over a single pulse cycle based on the single-pulse blood volume waveform and the single pulse blood pressure waveform; and

deriving a measure of cardiac work based on computing an area under the Pressure-Volume curve, and recording the measure of cardiac work in a cardiac work data series for the monitoring session; and

detecting acute cardiovascular events based on detecting changes in the measure of cardiac work during the monitoring session which meet one or more pre-defined alert criteria.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1.  A hemodynamic monitoring method (10), comprising:

receiving (12) from a wearable ultrasound sensor real-time Doppler ultrasound data and real time B-mode ultrasound data corresponding to an artery;

deriving (14) a continuous or real-time blood velocity signal by processing of the Doppler ultrasound data;

deriving (16), by processing of the B-mode ultrasound data, a real-time arterial diameter signal indicative of real time arterial diameter or a proxy thereof;

computing (18) a real-time blood flow or blood volume signal based on the arterial diameter signal and blood velocity signal;

computing (20) a continuous surrogate blood pressure signal based on applying a pre-determined blood pressure transfer function to the arterial diameter signal;

detecting (22) occurrence of one or more pre-defined acute hemodynamic events based on the continuous blood flow or blood volume signal and the continuous surrogate BP signal.

2. The method of claim 1, wherein the detecting one or more acute events comprises:

applying a signal decomposition to each of the blood flow/volume signal and the blood pressure signal to derive for each signal a respective set of extracted signal components; and

detecting the one or more acute events based on analysis of the extracted signal components for each signal.

3. The method of claim 1 or 2, wherein the signal decomposition comprises empirical mode decomposition (EMD).

4. The method of claim 2 or 3, wherein the detecting the one or more acute events based on analysis of the extracted signal components for each signal comprises:

selecting a subset of one or more of the signal components of each of the blood volume/flow signal and the blood pressure signal; and

detecting occurrence of one or more acute events based on one or more pre-defined detection criteria applied to the selected subset of signal components of the signals.

5. The method of claim 5, wherein the method comprises:

selecting a single signal component from the one or more of the signal components of each of the blood volume/flow signal and the blood pressure signal;

determining a phase difference between the selected signal component of the blood volume/flow signal and the selected component of the blood pressure signal; and

detecting occurrence of one or more acute events based on one or more pre-defined detection criteria applied to the phase difference.

6. The method of any preceding claim, wherein the pre-determined blood pressure transfer function takes the form:

$$p_{CCA}(t) = \delta D(t) + p_0$$

$$p_0 = p_d$$

$$\delta = \frac{MAP - p_d}{< D(t) >}$$

where pccv(t) is an estimated pressure signal in the common carotid artery, $D(t)$ is the derived arterial diameter signal, $p_d$ is a measured value of diastolic blood pressure, and $\delta$ is a scaling factor, wherein MAP is a measured value of mean arterial pressure and $< D(t) >$ is an average value of $D(t)$ over a single heart cycle.

7. The method of any preceding claim, wherein the detecting acute events comprises:

detecting suspected acute events by analysis of the extracted signal components of the continuous blood flow or blood volume signal and the continuous surrogate BP signal;

computing one or more cardiovascular indices using said signals for signal time periods corresponding to the suspected acute events;

confirming suspected acute events as confirmed acute events based on comparison of the computed indices against pre-defined confirmation criteria.

8. The method of claim 7, wherein the computed indices include one or more of:

heart rate (HR);
carotid flow time (CFT),
corrected carotid flow time (CFTc);
pulse contour (pressure);
velocity time integral (VTI);
phase shift between flow and pressure features (ΔPhase).

9. The method any preceding claim, wherein the method comprises,

at repeated time points during a patient monitoring session:

deriving a single-pulse blood volume waveform based on the computed real-time blood volume signal;

deriving a single-pulse blood pressure waveform based on the computed real-time blood pressure signal;

deriving a Pressure-Volume curve over a single pulse cycle based on the single-pulse blood volume waveform and the single

pulse blood pressure waveform; and

deriving a measure of cardiac work based on computing an area under the Pressure-Volume curve, and recording the measure of cardiac work in a cardiac work data series for the monitoring session; and

detecting acute cardiovascular events based on detecting changes in the measure of cardiac work during the monitoring session which meet one or more pre-defined alert criteria.

10. The method of claim 9,

wherein the deriving the single-pulse blood volume waveform based on the real-time blood volume signal comprises computing an average blood volume waveform from a plurality of pulse waveforms in the real-time blood volume signal; and/or
wherein the deriving the single-pulse blood pressure waveform based on the real-time blood pressure signal comprises computing an average blood pressure waveform from a plurality of pulse waveforms in the real-time blood pressure signal.

11. The method of claim 9 or 10, wherein the method further comprises estimating a phase offset between the single-pulse blood volume waveform and the single pulse blood pressure waveform by determining an average phase offset between peaks of a plurality of pulses in the real-time blood velocity signal and peaks of a plurality of pulses in the real-time arterial diameter signal.

12. The method of claim 11, wherein the method comprises time-aligning the single-pulse blood volume waveform and single-pulse blood pressure waveform based on the phase offset.

13. The method of any preceding claim, wherein the artery is a peripheral artery.

14. A computer program product comprising computer program code configured, when run on a processor, to cause the processor to perform a method in accordance with any preceding claim.

15. A system (30) comprising:

a wearable ultrasound sensor (54); and
a processing device (32), comprising an input/output (34) for connecting to the wearable ultrasound sensor, and one or more processors (36), configured to:

receive from the wearable ultrasound sensor real-time Doppler ultrasound data and real time B-mode ultrasound data corresponding to an artery;
derive a continuous or real-time blood velocity signal by processing of the Doppler ultrasound data;
derive, by processing of the B-mode ultrasound data, a real-time arterial diameter signal indicative of real time arterial diameter or a proxy thereof;
compute a real-time blood flow or blood volume signal based on the arterial diameter signal and blood velocity signal;
compute a continuous surrogate blood pressure signal based on applying a predetermined blood pressure transfer function to the arterial diameter signal;
detect acute hemodynamic events based on the continuous blood flow or blood volume signal and the continuous surrogate BP signal.

10 ↘

| Receive U/S data | ~ 12 |

↓

| Derive blood vel. signal | ~ 14 |

↓

| Derive diameter signal | ~ 16 |

↓

| Compute blood flow signal | ~ 18 |

↓

| Compute surrogate blood pressure WF | ~ 20 |

↓

| Detect acute hemo. events | ~ 22 |

## FIG. 1

FIG. 2

FIG. 3

FIG. 4

EP 4 649 892 A1

FIG. 5

FIG. 6

FIG. 7

FIG. 8

FIG. 9

EUROPEAN SEARCH REPORT

**Application Number**

EP 24 17 5594

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KENNY JON-ÉMILE S. ET AL: "The Doppler shock index measured by a wearable ultrasound patch accurately detects moderate-to-severe central hypovolemia during lower body negative pressure", JOURNAL OF THE AMERICAN COLLEGE OF EMERGENCY PHYSICIANS OPEN, vol. 2, no. 4, 1 August 2021 (2021-08-01), XP093211955, ISSN: 2688-1152, DOI: 10.1002/emp2.12533 Retrieved from the Internet: URL:https://onlinelibrary.wiley.com/doi/full-xml/10.1002/emp2.12533> * abstract; figures 2,4 * * page 3, left-hand column, paragraph 2 - page 6, left-hand column, paragraph 2 * ----- | 1-15 | INV. A61B8/04 A61B8/06 A61B8/08 A61B8/00 |
| A | EP 4 275 611 A1 (KONINKLIJKE PHILIPS NV [NL]) 15 November 2023 (2023-11-15) * paragraphs [0004], [0011] - [0021], [0030] - [0053], [0072]; figures 1-5 * ----- -/-- | 1-15 | TECHNICAL FIELDS SEARCHED (IPC) A61B |

The present search report has been drawn up for all claims

| Place of search | Date of completion of the search | Examiner |
|---|---|---|
| Munich | 14 October 2024 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 17 5594

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SEO JOOHYUN ET AL: "Non-Invasive Evaluation of a Carotid Arterial Pressure Waveform Using Motion-Tolerant Ultrasound Measurements During the Valsalva Maneuver", IEEE JOURNAL OF BIOMEDICAL AND HEALTH INFORMATICS, IEEE, PISCATAWAY, NJ, USA, vol. 25, no. 1, 18 May 2020 (2020-05-18), pages 163-174, XP011829426, ISSN: 2168-2194, DOI: 10.1109/JBHI.2020.2995344 [retrieved on 2021-01-04] * abstract; figures 1-5,8 * * page 163, left-hand column, paragraph 1 - page 166, right-hand column, paragraph 2 * * page 167, left-hand column, paragraph 5 - right-hand column, paragraph 5 * ----- | 1-15 | |
| A | CN 102 652 679 A (XIAOMENG TONG) 5 September 2012 (2012-09-05) * abstract * ----- | 1-15 | TECHNICAL FIELDS SEARCHED (IPC) |
| A | US 2021/386299 A1 (HOCKING GRANT [US]) 16 December 2021 (2021-12-16) * paragraphs [0053], [0056] - [0057], [0113]; claim 1; figures 1a-c,5 * ----- | 1-15 | |

The present search report has been drawn up for all claims

| Place of search | Date of completion of the search | Examiner |
|---|---|---|
| Munich | 14 October 2024 | Daoukou, Eleni |

EPO FORM 1503 03.82 (P04C01)

## ANNEX TO THE EUROPEAN SEARCH REPORT
## ON EUROPEAN PATENT APPLICATION NO.

EP 24 17 5594

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2024

| Patent document cited in search report | | Publication date | Patent family member(s) | | Publication date |
|---|---|---|---|---|---|
| EP 4275611 | A1 | 15-11-2023 | EP | 4275611 A1 | 15-11-2023 |
| | | | WO | 2023217565 A1 | 16-11-2023 |
| CN 102652679 | A | 05-09-2012 | NONE | | |
| US 2021386299 | A1 | 16-12-2021 | AU | 2021293550 A1 | 16-02-2023 |
| | | | CA | 3183010 A1 | 23-12-2021 |
| | | | EP | 4164477 A1 | 19-04-2023 |
| | | | US | 2021386299 A1 | 16-12-2021 |
| | | | WO | 2021257341 A1 | 23-12-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Turkish Journal of Emergency Medicine*, January 2020, vol. 20 (1)

- **FOLTS et al.** Coronary and Hemodynamic Effects of Temporary Acute Aortic Insufficiency in Intact Anesthetized Dogs. *Circulation Research.*, August 1974, vol. 35